# EUROPEAN PATENT APPLICATION

(11) **EP 2 803 378 A1**
(43) Date of publication of application: **19.11.2014**
(21) Application number: 14162271.2
(22) Date of filing: 28.03.2014
(51) Int. Cl.: A61M 5/315

(54) **Gasket for syringe**

(30) Priority: 15.05.2013 JP 2013103451
(71) Applicant: Sumitomo Rubber Industries, Ltd., Hyogo-ken (JP)
(72) Inventor: Nakano, Hiroaki, Kobe-shi, Hyogo-ken, 651-0072 (JP); Yao, Eiji, Kobe-shi, Hyogo-ken, 651-0072 (JP); Iwano, Shinya, Kobe-shi, Hyogo-ken, 651-0072 (JP); Ishida, Naoyuki, Kobe-shi, Hyogo-ken, 651-0072 (JP)
(74) Representative: Manitz, Finsterwald & Partner GbR

(57) **Abstract**

The present invention provides a gasket for syringes which can be stored for a long time without any chemical liquid leakage. The present invention relates to a gasket for syringes, including a film laminated to a surface of the gasket, the film having a liquid-contacting portion and a sliding portion, the liquid-contacting portion being thicker than the sliding portion. Preferably, the liquid-contacting portion has a thickness at least 1.2 times the thickness of the sliding portion.

## Description

### TECHNICAL FIELD

The present invention relates to a gasket for syringes. Specifically, the present invention relates to a gasket for syringes (prefilled syringes) including a film laminated to a surface of the gasket.

### BACKGROUND ART

Syringes pre-filled with a chemical liquid (prefilled syringes) are used in medical applications. Prefilled syringes eliminate the need to troublesomely transfer chemical liquids from other containers and thus are easy to use; besides, they prevent medical malpractice in transferring chemical liquids. Therefore, they are being increasingly used these days. Such prefilled syringes need to have the properties required of containers which are to be in contact with chemical liquids for a long period of time, unlike the conventional syringes (in the case of the conventional syringes, a chemical liquid is drawn from another container, such as a vial, immediately before use).

Materials generally used for gaskets for syringes include crosslinked rubbers with various additives for crosslinking. When these additives or pyrolysates thereof contact a chemical liquid, they may transfer into the chemical liquid. These transferred additives or the like may adversely affect the efficacy and the stability of the chemical liquid.

Meanwhile, gaskets are required to smoothly slide on a syringe barrel. However, gaskets made of a crosslinked rubber are generally too poor in sliding properties to be used. For this reason, silicon oil is applied to the barrel or gasket surface. Silicone oil, however, may adversely affect the efficacy and the stability of chemical liquids.

In this context, products called "laminated gaskets," which are rubber gaskets whose surface is laminated with a film having good sliding properties, are known (see Patent Literature 1). Covering the surface of a rubber gasket with a film prevents the components in the crosslinked rubber from transferring into a chemical liquid. Further, using a film with high sliding properties for the lamination ensures sliding properties without applying silicone oil. Such films with high sliding properties include ultrahigh molecular weight polyethylene and fluororesins. Especially, fluororesins are suitable because they have high sliding properties and are chemically stable. A widely used fluororesin is polytetrafluoroethylene (PTFE) because it has very high sliding properties and very high stability.

These films with high sliding properties are, however, insufficient in rubber elasticity and thus they inhibit the rubber elasticity of the crosslinked rubber inside the film. Rubber elasticity is an essential property of the gasket for forming a secure seal to the chemical liquid in a syringe. Insufficient rubber elasticity causes the problem of leakage of the chemical liquid from the syringe. In order not to inhibit the rubber elasticity of the inside, thin films are preferred. However, thin films tend not to sufficiently prevent the components in a crosslinked rubber from transferring into a chemical liquid. Moreover, in the case of PTFE films, which are typically formed by skiving, if the film has a small thickness, pinholes are more likely to occur in the film, and thus there is the problem that such a film with a pinhole is unable to prevent the components in a crosslinked rubber from transferring into a chemical liquid.

### CITATION LIST

### PATENT LITERATURE

Patent Literature 1: JP 2013-49236 A

### SUMMARY OF INVENTION

### TECHNICAL PROBLEM

The present invention aims to provide a gasket for syringes (prefilled syringes) which solves the above problems and can be stored for a long time without any chemical liquid leakage.

### SOLUTION TO PROBLEM

Accordingly, the present invention relates to a gasket for syringes, including a film laminated to a surface of the gasket, the film including a liquid-contacting portion and a sliding portion, and the liquid-contacting portion being thicker than the sliding portion.

The liquid-contacting portion preferably has a thickness at least 1.2 times the thickness of the sliding portion.

Preferably, the liquid-contacting portion has a thickness of 25 µm or larger, and the sliding portion has a thickness of smaller than 25 µm.

Preferably, the liquid-contacting portion has a thickness of 35 µm or larger, and the sliding portion has a thickness of 20 µm or smaller.

The film is preferably made of a fluororesin.

The fluororesin is preferably a polytetrafluoroethylene (PTFE) resin, a modified PTFE resin, or an ethylenetetrafluoroethylene (ETFE) copolymer.

### ADVANTAGEOUS EFFECTS OF INVENTION

In the gasket for syringes of the present invention, the liquid-contacting portion of the film laminated to the surface of the gasket is thicker than the sliding portion of the film. Thus, the gasket can be provided as a gasket for prefilled syringes which is less likely to adversely affect the chemical liquid inside the syringe and also is excellent in sealing properties.

### BRIEF DESCRIPTION OF DRAWINGS

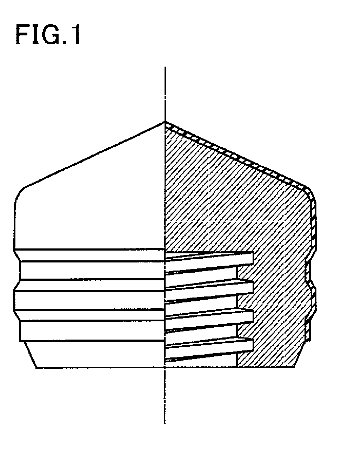

Fig. 1 is a partial cross-sectional view of each of the gaskets prepared in examples.

### DESCRIPTION OF EMBODIMENTS

The gasket for syringes of the present invention is characterized in that a film is laminated to the surface of the gasket, and the liquid-contacting portion of the film is thicker than the sliding portion of the film. The thickness of the liquid-contacting portion herein means the minimum thickness of the top surface portion to be in contact with a chemical liquid. The thickness of the sliding portion herein means the thickness of the maximum gasket diameter portion to be in contact with a barrel. By making the liquid-contacting portion of the film thicker than the sliding portion of the film, it is possible for the film to prevent the transfer of the components in the rubber while providing chemical liquid-sealing properties.

The liquid-contacting portion of the laminate film is suitably thick in terms of preventing the components in the crosslinked rubber from transferring into a chemical liquid. The sliding surface to be in contact with a barrel, on the other hand, serves as a chemical liquid-sealing surface during the storage of the prefilled syringe, and thus is suitably as thin as possible to ensure the rubber elasticity of the gasket. Since the gasket requires a laminate film on the surface thereof to ensure sliding properties, it is necessary that the liquid-contacting portion of the film should be thicker than the sliding portion.

The thickness of the liquid-contacting portion of the film is preferably at least 1.2 times, and more preferably at least 1.75 times the thickness of the sliding portion. If the thickness of the liquid-contacting portion is less than 1.2 times the thickness of the sliding portion, then there is no sufficient difference in thickness between the liquid-contacting portion and the sliding portion, which tends to make it impossible to simultaneously ensure the required thickness of the liquid-contacting portion and the required thinness of the sliding portion. The upper limit of the thickness of the liquid-contacting portion is not particularly limited, and the thickness is preferably at most 10 times, and more preferably at most 7 times the thickness of the sliding portion.

The thickness is not particularly limited, and may be appropriately selected according to the required properties of the gasket. For example, to securely prevent the transfer of the components in the rubber, the thickness of the entire film may be increased. To form a secure seal to a chemical liquid, the thickness of the entire film may be decreased.

The thicker the liquid-contacting portion is, the more easily the transfer of the components in the crosslinked rubber can be prevented. The thickness of the liquid-contacting portion is preferably 25 µm or larger, and more preferably 35 µm or larger. The upper limit thereof is not particularly limited, and the thickness is preferably 150 µm or smaller from the viewpoint of moldability and economic efficiency. If the thickness of the liquid-contacting portion is smaller than 25 µm, the transfer of the components in the crosslinked rubber tends not to be easily prevented.

A thinner sliding portion is more suitable. The thickness of the sliding portion is preferably smaller than 25 µm, and more preferably 20 µm or smaller. The lower limit thereof is not particularly limited, and the thickness is preferably 5 µm or larger. If the thickness of the sliding portion is larger than 25 µm, the sliding portion tends to inhibit the rubber elasticity of the inside crosslinked rubber, making it difficult to form a secure seal to the chemical liquid in a syringe. If the thickness of the sliding portion is smaller than 5 µm, the laminate film necessary for the gasket to provide sliding properties tends not to be uniformly provided on the gasket.

The kind of film is not particularly limited as long as it is capable of preventing the transfer of the components from the crosslinked rubber and has better sliding properties than rubber, i.e., has a smaller friction coefficient than rubber. Examples of the films include ultrahigh molecular weight polyethylene and fluororesins, which have track records in medical applications. Preferred among these are fluororesins because they are excellent in sliding properties as well as in surface chemical stability. Any of known fluororesins containing fluorine is usable. Examples thereof include polytetrafluoroethylene (PTFE), modified PTFEs (e.g. copolymers of tetrafluoroethylene and a small amount of a perfluoroalkoxide monomer), ethylene tetrafluoroethylene (ETFE) copolymers, and perfluoroalkyl vinyl ether (PFA) copolymers. PTFE and modified PTFEs are preferred because these are particularly excellent in both sliding properties and chemical stability. ETFE is also preferred because it is highly resistant to γ-radiation sterilization. The film may consist of a single layer, or may have a laminated structure, as long as it satisfies the thickness requirements described above.

The gasket base material may include any elastic material, and examples thereof include various rubber materials such as natural rubber, butyl rubber, isoprene rubber, polybutadiene rubber, styrene-butadiene rubber, silicone rubber, epichlorohydrin rubber, ethylene propylene rubber, and nitrile rubber; and various thermoplastic elastomers such as polyurethane-based, polyester-based, polyamide-based, olefin-based, and styrene-based thermoplastic elastomers. These elastic materials may be used alone or as a blend of two or more. Preferred among these are materials which become elastic when vulcanized. In terms of moldability, ethylene-propylene-diene rubber, polybutadiene rubber, and the like are preferred. In terms of gas permeation resistance, butyl rubber, chlorinated butyl rubber, brominated butyl rubber, and the like are preferred. In the case of vulcanizable materials, additives known in the rubber industry, such as a vulcanizing agent (e.g. sulfur) and a vulcanization accelerator, may be appropriately added.

The thickness of the film except for the liquid-contacting portion and the sliding portion is not particularly limited, and is preferably 5 to 150 µm although it may be appropriately selected from the viewpoint of moldability and economic efficiency.

Any known silicone oil or curable silicone oil may be applied to the inner surface of a barrel or the gasket surface to achieve higher sliding properties, as long as the oil does not adversely affect the chemical liquid inside the barrel.

The gasket of the present invention may be prepared as follows. Compounding materials at predetermined ratios are kneaded in an internal mixer, an open roll mill, or the like to provide a kneaded mixture. The kneaded mixture is sheeted in a calendar or a sheeting machine to provide an unvulcanized rubber sheet. Subsequently, a sheet having a certain weight and size is cut from the unvulcanized rubber sheet, and then the cut sheet and an inert film are stacked on a mold, and molded by vacuum press to provide a molded laminated gasket sheet.

Here, the inert film is used after the thickness of the portion corresponding to the liquid-contacting portion of the gasket is changed from that of the portion corresponding to the sliding portion of the gasket. Such a film whose thickness is partially changed may be prepared, for example, by forming a film from a dispersion of non-melting PTFE by casting while partially changing the number of laminations; or, alternatively, by pressing a melting ETFE film or the like between metal plates with thicknesses adjusted to change the thickness of the film.

The molding conditions are not particularly limited, and may be appropriately set. The molding temperature is preferably 155°C to 200°C, and more preferably 165°C to 180°C. The molding time is preferably 1 to 20 minutes, more preferably 3 to 15 minutes, and even more preferably 5 to 10 minutes.

Thereafter, unnecessary parts of the gasket molding are cut away and removed, and then washed, sterilized, dried, and checked for appearance. Thus, a finished gasket is obtained.

### EXAMPLES

In the following, the present invention is described in more detail with reference to, but not limited to, examples.

The materials used in examples are listed below.

### (Film)

Fluororesin dispersion: POLYFLON D210-C, a product of Daikin Industries, Ltd.
PTFE film: VALFLON, a product of Nippon Valqua Industries, Ltd. Modified PTFE film: VALFLON Ex1, a product of Nippon Valqua Industries, Ltd.
ETFE film: Fluon ETFE, a product of Asahi Glass Co., Ltd. (Gasket base material)
Unvulcanized rubber sheet: halogenated butyl rubber Cross-linking agent:
   2-di-n-butylamino-4,6-dimercapto-s-triazine (Zisnet DB, a product of Sankyo Kasei Co., Ltd.)

### Examples 1 to 7 and Comparative Examples 1 to 3

A fluororesin film shown in Table 1 and an unvulcanized rubber sheet were stacked and placed on a mold, and then molded in a vacuum press for 8 minutes at 180 °C and a treatment pressure of 20 MPa for vulcanization bonding. A partial cross-sectional view of the prepared gasket is shown in Fig. 1. The molding was performed such that the maximum diameter ϕ of the gasket was 6.60 mm. The fluororesin film was used after its surface was roughened by a method disclosed in JP 2012-36249 A (which is incorporated by reference in the entirety), that is, by irradiating the film surface with an ion beam.

### PTFE film

In the formation, an aqueous PTFE dispersion was applied to one side of a polyimide sheet having a thickness of 0. 1 mm such that the thickness of the liquid-contacting portion of the resulting fluororesin film was changed from that of the sliding portion of the fluororesin film. After the sheet was subjected to preliminary heating at 90 °C for 5 min. to evaporate and remove water, the resulting sheet was heated at 350°C for 5 minutes for baking of a PTFE film. This operation was repeated several times to prepare a film having a desired thickness.

Here, to partially change the thickness, the dispersion was applied, or not applied, to the region corresponding to the maximum diameter portion of the molded gasket. In this manner, a PTFE film having portions of different thicknesses was obtained.

### Modified PTFE film

A desired film having portions of different thicknesses was obtained by partially applying, or not partially applying, an aqueous modified PTFE dispersion in the same manner as for the PTFE film.

### ETFE film

The above-mentioned ETFE film was heated to 300°C while it was pressed between two metal plates with adjusted thicknesses. In this manner, a film whose thickness was partially changed was obtained.

In the molding, each film having portions of different thicknesses such that the thickness of the liquid-contacting portion of the fluororesin film was changed from that of the sliding portion of the fluororesin film was placed on the corresponding mold part. Molding was then carried out to provide a gasket in which the liquid-contacting portion and the sliding portion of the film had different thicknesses. Each gasket was subjected to the following tests.

### <Measurement of film thickness>

Each molded gasket was cut and the cross-sections were observed with an electron scanning microscope (S-3400N, a product of Hitachi High-Technologies Corporation) at 150 X magnification to determine the thickness of the film. The thickness of the liquid-contacting portion was determined by measuring the film at the center of the top surface of the gasket. The thickness of the sliding portion was determined by measuring the maximum gasket diameter portion of the film. The cross-sections of five gaskets were observed, and the average of the five measurements is shown in Table 1.

### <Pinhole inspection>

The top surface of each molded gasket was observed with a video microscope (DVM5000, a product of Leica Microsystems) equipped with a 300X objective lens. The number of pinholes having a diameter of 10 µm or larger was then counted. Twenty gaskets were used to observe the top surface in five points (field of view), and the number of pinholes counted is shown in Table 1. Those with not more than two pinholes are evaluated as good.

### <Chemical liquid sealing properties>

Each molded gasket was inserted into a barrel (made of a chlorolefin resin, inner diameter ϕ: 6. 35 mm). Subsequently, the barrel was filled with a test fluid (a mixture of water and 0.2 g/L of pigment (methylene blue, a product of Sigma-Aldrich Japan K.K.)) and the opposite side of the barrel was capped. The barrel was allowed to stand still at 70°C for one week. Thereafter, the barrel was observed with a video microscope (DVM5000, a product of Leica Microsystems) equipped with a'50X objective lens to check the presence or absence of liquid leakage. Twenty gaskets were used in the observation. Those in which the test fluid had passed through the maximum gasket diameter portion were rated as having liquid leakage, and the number of gaskets with liquid leakage is shown in Table 1. Those with not more than two liquid leakages are evaluated as good.

**[Table 1]**

| Example number | | | Example | | | | | | | Comparative Example | | |
|---|---|---|---|---|---|---|---|---|---|---|---|---|
| | | | 1 | 2 | 3 | 4 | 5 | 6 | 7 | 1 | 2 | 3 |
| Film | Liquid-contacting portion | Type | PTFE | PTFE | PTFE | PTFE | PTFE | Modified PTFE | ETFE | PTFE | PTFE | Modified PTFE |
| | | Thickness (mm) | 0.08 | 0.06 | 0.07 | 0.05 | 0.15 | 0.07 | 0.06 | 0.08 | 0.04 | 0.04 |
| | Sliding portion | Type | PTFE | PTFE | PTFE | PTFE | PTFE | Modified PTFE | ETFE | PTFE | PTFE | Modified PTFE |
| | | Thickness (mm) | 0.06 | 0.06 | 0.05 | 0.04 | 0.04 | 0.06 | 0.05 | 0.12 | 0.08 | 0.08 |
| Film thickness in product | Top surface portion (*µ*m) | | 43 | 30 | 36 | 25 | 98 | 35 | 31 | 47 | 19 | 19 |
| | Sliding portion (*µ*m) | | 25 | 24 | 19 | 14 | 15 | 20 | 20 | 63 | 31 | 32 |
| | Top surface portion/sliding portion | | 1.7 | 1.3 | 1.9 | 1.8 | 6.5 | 1.8 | 1.6 | 0.7 | 0.6 | 0.6 |
| Test results | Pinhole inspection | | 0 | 1 | 0 | 2 | 0 | 0 | 0 | 0 | 6 | 9 |
| | Liquid leakage | | 1 | 1 | 0 | 0 | 0 | 0 | 1 | 7 | 5 | 4 |

In the gaskets of Examples 1 to 7, in which the liquid-contacting portion of the film was thicker than the sliding portion of the film, a few pinholes were formed and thus only a small amount of components eluted from the crosslinked rubber; besides, good sealing properties were achieved. In contrast, in the gaskets of Comparative Examples 1 to 3, in which the liquid-contacting portion of the film was thinner than the sliding portion of the film, many pinholes were formed and thus a large amount of components eluted from the crosslinked rubber, or the chemical liquid-sealing properties were poor. Thus, the gaskets of the comparative examples were unable to simultaneously achieve these properties.

## Claims

1. A gasket for syringes, comprising a film laminated to a surface of the gasket,
the film comprising a liquid-contacting portion and a sliding portion, and
the liquid-contacting portion being thicker than the sliding portion.

2. The gasket for syringes according to claim 1,
wherein the liquid-contacting portion has a thickness at least 1.2 times the thickness of the sliding portion.

3. The gasket for syringes according to claim 1 or 2,
wherein the liquid-contacting portion has a thickness of 25 µm or larger, and the sliding portion has a thickness of smaller than 25 µm.

4. The gasket for syringes according to claim 3,
wherein the liquid-contacting portion has a thickness of 35 µm or larger, and the sliding portion has a thickness of 20 µm or smaller.

5. The gasket for syringes according to any one of claims 1 to 4,
wherein the film is made of a fluororesin.

6. The gasket for syringes according to claim 5,
wherein the fluororesin is a polytetrafluoroethylene (PTFE) resin, a modified PTFE resin, or an ethylenetetrafluoroethylene (ETFE) copolymer.
